Europäisches Patentamt

European Patent Office

Office européen des brevets

(11) Publication number: **0 089 854**
**B1**

# EUROPEAN PATENT SPECIFICATION

(45) Date of publication of patent specification: **07.12.88**

(51) Int. Cl.⁴: **A 61 K 39/12, A 61 K 39/245**

(21) Application number: **83301608.2**

(22) Date of filing: **23.03.83**

(54) **Vaccine against DNA viruses.**

(30) Priority: **24.03.82 GB 8208650**

(43) Date of publication of application:
**28.09.83 Bulletin 83/39**

(45) Publication of the grant of the patent:
**07.12.88 Bulletin 88/49**

(84) Designated Contracting States:
**AT BE CH DE FR GB IT LI LU NL SE**

(56) References cited:
**EP-A-0 001 365**
**EP-A-0 048 201**
**GB-A-2 037 165**

**LA RECHERCHE, vol. 8, no. 82, October 1977,
"Nouvelles internationales", p. 873**

(73) Proprietor: **THE UNIVERSITY OF BIRMINGHAM
Chancellor's Court P.O. Box 363
Birmingham B15 2TT (GB)**

(72) Inventor: **Skinner, Gorden Robert Bruce
58 Seven Stars Road
Solihull (GB)**
Inventor: **Buchan, Alexander
278 Harborne Park Road
Harborne Birmingham (GB)**

(74) Representative: **Cuddon, George Desmond et al
MARKS & CLERK Alpha Tower Suffolk Street
Queensway
Birmingham B1 1TT (GB)**

Courier Press, Leamington Spa, England.

## Description

It has been proposed, in published U.K. Patent Application 2037165A, to prepare a vaccine against a viral illness by infecting cells with the virus, treating the infected cell material with a detergent to part the infected cell nuclei from their cytoplasmic fraction, and reacting the cytoplasmic fraction with antibodies to the virus, to form immunocomplexes with the virus antigens, these immunocomplexes providing the required vaccine.

It has hitherto been accepteed that in addition to parting the infected cell nuclei from the cytoplasmic fraction, the detergent inactivates all of the residual virus, and in addition inactivates all of the residual virus nucleocapsids. It has not therefore been the practice to treat the cytoplasmic fraction chemically before formation of the immunocomplexes.

Moreover, it has been proposed, for example in EP—A—48201, to prepare a vaccine against a DNA virus, for example Herpes simplex virus, by infecting cells with the virus, extracting virus antigens from the infected cell material by means of urea, and precipitating the antigens so as to remove unwanted components. It is a disadvantage of the prior art extraction step that it will necessarily disrupt the cell nuclei in which over 90% of virus DNA is present. In prior art methods the extract is treated with DNase which will degrade the intact virus DNA. The treated extract will contain DNase and fractionated virus DNA, both of which are unacceptable components of a vaccine. The prior art treated extract is therefore fractionated chromatographically to separate the required immunogenic fraction from the DNase and residual DNA, as well as from other unwanted components.

It is a disadvantage of the prior art process that chromatographic separation results in a very low yield of vaccine from a given starting quantity of infected cell material.

It is an object of the invention to provide a method of preparing a vaccine against a DNA virus, in which an improved yield of substantially pure virus antigen is obtained without complex processing. The present invention obtains this result by initial retrieval of the maximum amount of virus antigen from an infected cell material, while excluding as much as possible of the virus particles, virus DNA and cell proteins, all of which would require later removal.

Initially therefore the present invention includes the step of separating the cytoplasmic fraction from the nuclei of the infected cell material. Additionally the cytoplasmic fraction is chemically treated to stabilise the polypeptide chains of the proteins of the cytoplasmic fraction, which has been found not only to improve the immunogenic quality of the vaccine but also to inactivate residual virus DNA and nucleocapsids, which may be present at this stage. As a result of these steps precipitation of the virus antigenic proteins may be simply effected by addition of a polar organic solvent which increases protein-protein interaction by lowering the dielectric constant of a water-solvent mixture in which the proteins are present.

According to the invention a method of preparing a vaccine against a DNA virus includes the steps of:—

(i) infecting a living cell sample with the virus;

(ii) incubating the infected cell;

(iii) separating by means of a detergent the nuclei of the infected cell material from the cytoplasmic fraction thereof;

(iv) stabilising by chemical reaction, the polypeptide chains in said cytoplasmic fraction; and

(v) forming a precipitate which includes the virus antigens in the cytoplasmic fractions.

Embodiments of the invention will now be described by way of example only and with reference to a vaccine against a Herpes simplex virus strain whose characteristics are shown by the accompanying drawings, in which:

Figure 1 shows the electrophoretic distribution of the molecular weights of characteristic proteins of the virus; and

Figure 2 is an expanded electrophoretic distribution of part of Figure 1.

Herpes simplex virus is a relatively large virus consisting of (1) a nucleoprotein 'core' measuring 75 mu in diameter containing double-stranded deoxyribonucleic acid (DNA); (2) a capsid with icosahedral symmetry consisting of 162 capsomeres and, in a certain proportion of particles, (3) a glycoprotein-containing lipo-protein envelope.

An infected cell material is incubated using MRC5 cells, a human embryonic cell line. MRC5 cells are at present the preferred cells, and in some cases their use is mandatory. These cells are, however, slow growing, and recently the possibility has been considered that vaccines obtained by their use could result in a vaccinated person developing an immune reaction to other human tissue, thereby introducing the possibility of rejection of any subsequent tissue transplant. Embodiments of the processes presently described include steps by means of which the cell proteins may be removed and will thereby no longer be able to elicit an immune response. Additionally, removal of the cell protein should overcome objections to the use of cell lines other than MRC5.

For the described example the MRC5 cells are obtained from the National Institute for Biological Standards and Controls, Hampstead, England. These cells are cultivated in Eagle's medium supplemented with 10% foetel or newborn calf serum and 10% tryptose phosphate broth to growth in rotating Winchester bottles (2,500 ml) which normally entailed 3—4 passages. The foetal calf serum is obtainable from Sera-Lab. Plasma Laboratory Limited, Crawley Down, Sussex, England. Tryptose phosphate broth is obtainable from Difco Laboratories, West Molesey, Surrey, England, and sterilized by autoclaving. Aliquots of the cultivated cells are stored in a glycerol-containing medium at −70°C for subsequent use.

The cells are allowed to reach near confluence as sheets within 2,500 ml rotating Winchester bottles. Each cell sheet is subsequently washed with prewarmed Eagle's medium to remove the foetal calf serum. The cells are maintained in a serum-deprived condition for 24 hours. Serum deprivation will tend to result in a significant reduction in the level of virus antigens. Though serum deprivation has previously been considered necessary in order to reduce the level of calf serum in the resulting vaccine, it is envisaged that at least some of the steps described hereinafter, of precipitating virus proteins from the infected cell extract, may allow the serum deprivation step to be omitted entirely, whereby the virus antigen yield may be substantially increased.

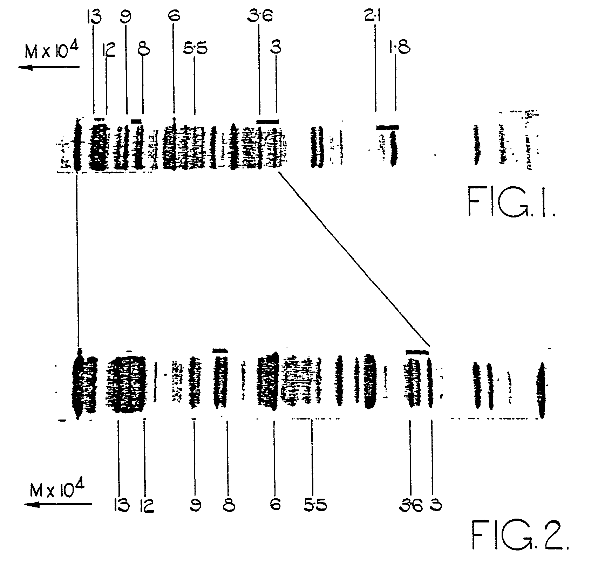

After the 24 hours of serum deprivation the cells are washed with pre-warmed Eagle's medium and infected with Herpes simplex virus type 1 (HSV 1). The virus used is the Troisbell strain developed in the Department of Medical Microbiology of the University of Birmingham, England. The virus was isolated from an oral cold sore of an otherwise healthy middle-aged subject, and identified as HSV 1 by neutralisation kinetics, agar gel diffusion tests, and by polypeptide analysis using polyacrylamide gel electrophoresis. The patterns obtained by gel electrophoresis are shown in Figure 1 and 2 and will be described in detail hereafter. The strain was isolated in human embryonic lung tissue and subsequently cloned three times by limit dilution in a preparation of the MRC5 cells previously referred to. This preparation comprised MRC5 cells cultivated in Eagle's medium containing gamma-irradiated foetel calf serum. The cloned virus was isolated and identified as HSV1 by the methods indicated above, which were used for the original identification.

The Troisbell virus strain is added to the serum-deprived cells at a multiplicity of 5 plaque-forming units per cell. Virus absorption is continued for 1 hour at 37°C following which the cells are again washed with pre-warmed Eagle's medium and re-incubated.

It has been found that after 24 hours of incubation the cytophatic level of the incubated material is 100%. At this stage each cell sheet is again washed with Eagle's medium and removed from its Winchester bottle. The cells are then suspensed in phosphate buffered saline to a concentration of $4 \times 10^7$ ml. Nonidet® P40 detergent, obtainable from BDH Chemicals Ld. of Poole, England, and having the product designation 56009, is added, to a final concentration of 1% by volume, and maintained thus at room temperature. The resulting preparation is centrifuged at about 650 g for 10 minutes and the supernatant, which is the cytoplasmic fraction of the preparation, is collected, the remaining material being discarded. The action of the Nonidet P40 detergent is to part the nuclei of the cells from their cytoplasm and to strip important antigenic proteins from the enveloped virus particle. The Nonidet also reduces, but may not totally eliminate, the infectivity of the virus particles. The supernatant thus at this stage contains virus antigenic proteins and virus particles, human embryonic cell proteins, and proteins from the foetal calf serum, as well as small amounts of extra-particulate virus DNA, together with Nonidet detergent.

The polypeptide chains in the cytoplasmic fraction are stabilized using a known fixing agent such as formaldehyde or glutaraldehxde, to a final concentration of 0.04%. In addition to fixing the polypeptide chains this step inactivates any residual virus and cell DNA and reacts with the virus particles to inactivate any residual virus nucleocapsids.

The resulting fraction is then centrifuged over a cushion of sucrose at a concentration of 20% W/V for 5 hours at $10^5$g, in a wide bucket of 40 ml volume. The sucrose is Grade 1 and is obtainable from Sigma Biochemicals Poole, England, under the product designation 59378. The 4 ml top fraction in each centrifuge container comprises a purified cytoplasmic fraction which has been found to contain no virus particles or virus nucleic acid, and to consist of virus proteins, together with cell and serum proteins, as well as the formaldehyde, sucrose and detergent previously added.

The purified cytoplasmic fraction is then subjected to a process which precipitates the virus protein from the fraction, either alone or in combination with antibodies to that protein. This precipitation may be accomplished by the following steps, or combinations of steps:

(i) The purified cytoplasmic fraction is passed through an activated sepharose column to which antibodies to the MRC5 cell proteins are covalently bound. The sepharose column may also include antibodies to the foetal calf serum in which the MRC5 cells are incubated. Alternatively, the top fraction may be passed through successive activated sepharose columns, to remove any proteions other than the virus antigens.

The foregoing step or steps, cause the unwanted proteins to form antigen/antibody complexes which remain within the column and, as indicated above, enables cell lines other than MRC5 to be used for vaccine preparation and also enables the serum deprivation step to be reduced in duration, or possibly to be eliminated.

(ii) Preferably after removal of the unwanted proteins, aluminium hydroxide adjuvant is added to the preparation and adsorbs the virus antigens as a washable suspension. The resulting mixture is agitated at room temperature for one hour and subsequently centrifuged at low speed to separate the antigen/adjuvant suspension. The supernatant, containing the Nonidet detergent fixing agent (e.g. formaldehyde) and sucrose is discarded and the separated suspension is washed in sterile phosphate buffered saline and forms the required vaccine.

In addition to enabling the virus antigens to be selected out, the aluminium hydroxide suspension permits washing to remove all traces of the supernatant. The volume of aluminium hydroxide required is a small proportion of the volume of the preparation which is added to it. Large amounts of the preparation may therefore be treated in a container which is not substantially larger than the volume of the preparation currently passing through this step.

The aluminium hydroxide acts as an adjuvant which enhances the effectiveness of the resulting vaccine, and the suspension is readily stored.

(iii) Alternatively, aluminium hydroxide adjuvant is added directly to the purified cytoplasmic fraction, to adsorb the virus antigens and proteins from the MRC5 cells as a washable suspension, the mixture being treated as in step (ii) above, to provide the vaccine.

(iv) The purified cytoplasmic fraction is alternatively reacted with antibodies to the viral proteins, to provide an antigen/antibody precipitate which is separated from the remainder of the material by centrifugration. The separated precipitate is washed in phosphate-buffered saline to remove all traces of Nonidet, fixing agent, sucrose and MRC5 cell protein, this precipitate forming the required vaccine. If desired aluminium hydroxide may be added to the precipiate, as an adjuvant only.

(v) In another alternative step a suitable organic solvent which reacts with water in preference to the proteins in the fixed cytoplasmic fraction, is added to that fraction and precipitates the proteins therein. Acetone, ethanol and methanol are examples of such solvents and are added at temperatures between 0°C and −30°C at a ratio of 10 parts of solvents to 1 part of water in the cytoplasmic fraction. Preferably, the solvent temperature is between −10°C and −20°C. The precipitate is separated by centrifugation and the remaining solvent on the precipitate is evaporated off. The precipitated proteins are then either re-suspended .in phosphate-buffered saline or added to aluminium hydroxide adjuant.

Since this step precipitates all the proteins in the purified cytoplasmic fraction, it is necessary that the proteins from the calf serum used at an earlier stage have previously been removed. If an organic solvent is used to effect protein precipitation, it is therefore necessary to have subjected the MRC5 cells to serum deprivation, as described above, before infection with the virus.

The electrophoresis gel pattern of the Troisbell HSV1 virus used for preparation of the vaccine are shown in Figures 1 and 2, in which moleculare weights M are shown as values ×10$^4$, and in which Figure 2 is an expansion of part of Figure 1, obtained by the use of a different gel. In addition to identifying the virus strain as HSV1, by virtue of molecular weight patterns which are common to all HSV1 strains, these gel patterns exhibit characteristics which will readily be recognised by one skilled in the art as being unique, and will enable identification of the Troisbell strain. Particularly characteristic of the Troisbell virus are the protein groups at molecular weights between 8×10$^4$ and 9×10$^4$, and between 3×10$^4$ and 3.6×10$^4$. Moreover these groupings, taken in conjunction with groupings between 1.8×10$^4$ and 2.1×10$^4$, between 5.5×10$^4$ and 6×10$^4$ and between 12×10$^4$ and 13×10$^4$, define a unique pattern which will enable identification of the Troisbell virus, vaccines derived therefrom, and of antibodies produced by vaccinated subjects. Use of this vaccine will thereby render it possible to determine whether the presence in a subject of antibodies to HSV1 is attributable to vaccination or to infection.

The Troisbell virus strain is held by the Department of Medical Microbiology, The University of Birmingham, Birmingham, England and is available on application on the same terms as those laid down by the Budapest Treaty.

A sample of the Troisbell virus strain has also been deposited at the American Type Culture Collection, Rockville, Maryland, U.S.A., and at the Collection National de Cultures de Micro-organismes in France.

510 patients have been treated with vaccine prepared according to the invention, without adverse effect. The tested vaccines were prepared by methods including the precipitation steps (iii) or (iv) or (v) described above.

Treatment of subjects with vaccine according to the invention has been found to prevent infection with herpes simplex virus type 2 (HSV2), the agent of genital herpes, and also to mitigate the recurrence of attacks in infected persons. According to established authorities, without vaccination 75% of the sexual partners of HSV2 sufferers develop the disease within one year. Tests with vaccines according to the invention have shown that less than 1% of previously unaffected persons who have been vaccinated develop genital herpes even after prolonged exposure.

Additionally, treatment by the vaccine of persons who have already suffered one attack of genital herpes has been found to result in a recurrence of an attack in only 25% of cases in a two year period after vaccination.

HSV2 is now recognised as a major health problem in some countries. It is estimated to affect 30% of the sexually active population in the U.S.A., and is increasing annually by between 250,000 and 500,000 cases. HSV2 can, if contracted at a late stage in pregnancy, have serious effect on the foetus. Additionally, infection with HSV2 has been shown to have a statistical connection with the incidence of cervical cancer.

The present invention provides a method of preparing a vaccine against HSV2, and in one of its aspects relates to a specific vaccine prepared from a virus strain which is itself believed to be particularly pure and safe.

**Claims**

1. A method of preparing a vaccine against a DNA virus, said method including infecting cells with said virus, incubating the infected cells and precipitating virus antigens from the infected cells, said virus antigens providing the active constituent of the vaccine, characterised in that before precipitation of said virus antigens the nuclei of said cells are separated by means of a detergent from a cell components which includes the cell cytoplasmic fraction, and said separated component is contacted with a fixing agent which stabilises the viral antigenic proteins in said cytoplasmic fraction by reacting with their polypeptide chains.

2. A method as claimed in Claim 1 in which said component includes antigenic proteins from the envelopes of particles of said virus.

3. A method as claimed in Claim 1 or Claim 2 in which separation of said cell nuclei is effected by a non-ionic detergent.

4. A method as claimed in any preceding claim in which said component is centrifuged over a sucrose cushion after stabilisation of said proteins and before precipitation of said antigens.

5. A method as claimed in any preceding claim in which said cells are subjected to serum deprivation before infection with said virus.

6. A method as claimed in any preceding claim in which precipitation of the virus antigens is effected by addition to said cell component of a polar organic solvent.

7. A method as claimed in Claim 6 in which said organic solvent, is selected from acetone, ethanol and methanol.

8. A method as claimed in Claim 6 or Claim 7 in which the temperature of said solvent is between 0°C and −30°C.

9. A method as claimed in any of Claims 1 to 5 in which precipitation of the virus antigens is effected by reacting said cell component with antibodies to said virus antigens and retaining the antigen-antibody complex so formed.

10. A method as claimed in any of claims 1 to 5 in which precipitation of the virus antigens is effected by reacting said cell component with antibodies to the proteins in said cells, separating and discarding the antigen-antibody complex so formed, and precipitating the remaining proteins.

11. A method as claimed in Claim 10 in which precipitation of said remaining proteins is effected by addition of aluminium hydroxide to said component after said antigen-antibody complex has been discarded.

12. A method as claimed in any of Claims 1 to 5 in which said precipitation of the virus antigens is effected by addition of aluminium hydroxide to said component after stabilisation of the viral antigenic proteins therein.

13. A method as claimed in any preceding claim in which said virus is a Herpes Simplex virus.

14. A method as claimed in Claim 13 in which said virus is characterised by the presence of proteins of the molecular weights substantially as indicated in Figures 1 and 2.

15. A vaccine against a DNA virus, said vaccine including substantially all of the virus antigenic proteins which are present in the cytoplasmic fractions of cells infected with the virus, the polypeptide chains of said antigenic proteins being stabilised.

**Patentansprüche**

1. Verfahren zur Herstellung eines Vakzins gegen ein DNA-Virus durch Infizieren von Zellen mit dem Virus, Inkubieren der infizierten Zellen und Ausfällen der Virus-Antigene aus den infizierten Zellen, wobei die Virus-Antigene den aktiven Bestandteil des Vakzins zur Verfügung stellen, dadurch gekennzeichnet, daß vor der Ausfällung der Virus-Antigene die Kerne der Zellen mit einem Reinigungsmittel von einer Zellkomponente, die die Zellzytoplasmafraktion umfaßt, abgetrennt werden und die abgetrennte Komponente mit einem Fixiermittel, das die viralen Antigenproteine in der Zytoplasmafraktion durch Reaktion mit ihren Polypeptidketten fixiert, kontaktiert wird.

2. Verfahren nach Anspruch 1, in dem die Komponente bie Antigenproteine aus den Partel umhüllungen des Virus enthält.

3. Verfahren nach Anspruch 1 oder 2, in der die Trennung der Zellkerne mit einem nichtionischen Reinigungsmittel durchgeführt wird.

4. Verfahren nach einem der vorsteheen Ansprüche, in dem die Komponente über einen Saccharosepuffer nach Stabilisierung der Proteine und vor Ausfällung der Antigene zentrifugiert wird.

5. Verfahren nach einem der vorstehenden Ansprüche, in dem den Zellen vor der Infektion mit dem Virus das Serum entzogen wird.

6. Verfahren nach einem der vorstehenden Ansprüche, in dem die Ausfällung der Virur-Antigene durch Zugabe eines polaren organischen Lösungsmittels zur Zellkomponente durchgeführt wird.

7. Verfahren nach Anspruch 6, in dem als organisches Lösungsmittel Aceton, Ethanol und Methanol gewählt wird.

8. Verfahren nach Anspruch 6 oder 7, in dem das Lösungsmittel mit einer Temperatur von 0 bis −30°C zugegeben wird.

9. Verfahren nach einem der Ansprüche 1 bis 5, in dem das Ausfällen der Virus-Antigene durch Umsetzen der Zellkomponente mit Antikörper zu den Virus-Antigenen und Zurückbehalten des so gebildeten Antigen-Antikörper-Komplexes durchgeführt wird.

10. Verfahren nach einem der Ansprüche 1 bis 5, in dem die Ausfällung der Virus-Antigene durch Umsetzen der Zellkomponente mit Antikörper zu den Proteinen in den Zellen, Abtrennen und Verwerfen des so gebildeten Antigen-Antikörper-Kompexes und Ausfällen der zurückbleibenden Proteine durchgeführt wird.

11. Verfahren nach Anspruch 10, in dem die Ausfällung der zurückbleibenden Proteine durch Zugabe von Aluminiumhydroxid zu der Komponente nach dem Verwerfen des Antigen-Antikörper-Komplexes durchgeführt wird.

12. Verfahren nach einem der Ansprüche 1 bis 5, in dem die Ausfällung der Virus-Antigene durch Zugabe von Aluminiumhydroxid zu der Komponente nach Stabilisierung der darin enthalten viralen Antigenproteine durchgeführt wird.

13. Verfahren nach einem der vorstehenden Ansprüche, in dem als Virus Herpes-Simplex-Virus verwendet wird.

14. Verfahren nach Anspruch 13, in dem das Virus durch Anwesentheit von Proteinen mit im wesentlichen Molekularmassen, wie sie in Fig. 1 und 2 dargestellt sind, charakterisiert ist.

15. Vakzin gegen ein DNA-Virus mit im wesentlichen all den antigenen Virusproteinen, die in der Zytoplasmafraktion von mit dem Virus infizierten Zellen vorhanden sind, wobei die Polypepidketten der Antigenproteine stabilisiert worden sind.

**Revendications**

1. Procédé pour préparer un vaccin contre un virus à ADN, lequel procédé comprend l'infection de cellules avec ledit virus, l'incubation des cellules infectées et la précipitation des antigènes viraux des cellules infectées, lesdits antigènes viraux fournissant le constituant actif du vaccin, caractérisé en ce qu'avant la précipitation desdits antigènes viraux, on séparé à l'aide d'un détergent les noyaux desdites cellules d'avec un composant cellulaire qui comprend la fraction cytoplasmique cellulaire, et on met ledit composant séparé au contact d'un agent fixant qui stabilise les protéines antigéniques virales dans ladite fraction cytoplasmique par réaction avec leurs chaînes polypeptidiques.

2. Procédé selon la revendication 1, dans lequel ledit composant comprend les protéines antigéniques des enveloppes des particules dudit virus.

3. Procédé selon la revendication 1 ou 2, dans lequel la séparation desdits noyaux cellulaires est effectuée avec un détergent non ionique.

4. Procédé selon l'une quelconque des revendications précédentes, dans lequel ledit composant est centrifugé sur un coussin de saccharose après stabilisation desdites protéines et avant précipitation desdits antigènes.

5. Procédé selon l'une quelconque des revendications précédentes, dans lequel lesdites cellules sont soumises à une restriction de sérum avant l'infection avec ledit virus.

6. Procédé selon l'une quelconque des revendications précédentes, dans lequel la précipitation des antigènes viraux est effectuée par addition d'un solvant organique polaire audit composant cellulaire.

7. Procédé selon la revendication 6 dans lequel ledit solvant est choisi parmi l'acétone, l'éthanol et le méthanol.

8. Procédé selon la revendication 6 ou 7, dans lequel la température du solvant est entre 0°C et −30°C.

9. Procédé selon l'une quelconque des revendications 1 à 5, dans lequel ladite précipitation des antigènes viraux est effectuée par réaction dudit composant cellulaire avec des anticorps contre lesdits antigènes viraux et conservation du complexe anticorps-antigène ainsi formé.

10. Procédé selon l'une quelconque des revendications 1 à 5, dans lequel la précipitation des antigènes viraux est effectuée par réaction dudit composant cellulaire avec des anticorps contre les protéines desdites cellules, séparation et rejet du complexe antigène-anticorps ainsi formé et précipitation des protéines restantes.

11. Procédé selon la revendication 10, dans lequel la précipitation desdites protéines restantes est effectuée par addition d'hydroxyde d'aluminium audit composant après rejet dudit complexe antigène-anticorps.

12. Procédé selon l'une quelconque des revendications 1 à 5, dans lequel ladite précipitation des antigènes viraux est effectuée par addition d'hydroxyde d'aluminium audit composant après stabilisation de ses protéines antigèniques virales.

13. Procédé selon l'une quelconque des revenducations précédentes, dans lequel ledit virus est un Herpès simplex virus.

14. Procédé selon la revendication 13, dans lequel ledit virus est caractérisé par la présence de protéines ayant des poids moléculaires essentiellement comme indiqué par les figures 1 et 2.

15. Vaccin contre un virus à ADN, ledit vaccin comprenant pratiquement la totalité des protéines antigénique virales qui sont présentes dans les fractions cytoplasmiques de cellules infectées par le virus, les chaînes polypeptidiques desdites protéines antigéniques étant stabilisées.

FIG.I.

FIG.2.